# EUROPÄISCHE PATENTANMELDUNG

(11) **EP 1 362 840 A1**
(43) Veröffentlichungstag der Anmeldung: **19.11.2003**
(21) Anmeldenummer: 03008690.4
(22) Anmeldetag: 16.04.2003
(51) Int. Cl.: C07C 51/34

(54) **Verfahren zur Herrstellung von aromatischen und heteroaromatischen Carbonsäuren durch katalytische Ozonolyse**

(30) Priorität: 15.05.2002 AT 7392002
(71) Anmelder: DSM Fine Chemicals Austria Nfg GmbH & Co KG, 4021 Linz (AT)
(72) Erfinder: Jary, Walther, 4853 Steinbach a. Attersee (AT); Pöchlauer, Peter, 4040 Linz (AT); Ganglberger, Thorsten, 4020 Linz (AT)
(74) Vertreter: Lindinger, Ingrid

(57) **Zusammenfassung**

Verfahren zur katalytischen Oxidation von alkylaromatischen Verbindungen der Formel (I)

Ar-CH₃

in der Ar einen gegebenenfalls substituierten, aromatischen oder heteroaromatischen 5er- oder 6er-Ring oder ein Ringsystem mit bis zu 20 C-Atomen bedeutet, wobei Ar gegebenenfalls mit einer C₁-C₆-Alkylgruppe, bei der bis zu 2 C-Atome durch ein Heteroatom ersetzt werden können, anelliert sein kann,

zu den korrespondierenden aromatischen oder heteroaromatischen Carbonsäuren, in einem Lösungsmittel mit Ozon in Gegenwart eines Übergangsmetallkatalysators und gegebenenfalls in Gegenwart einer Säure bei einer Temperatur zwischen - 70 °C und +70°C.

## Beschreibung

Aromatische und heteroaromatische Carbonsäuren, wie etwa Biphenylcarbonsäuren, Bipyridylcarbonsäuren, verschieden substituierte Benzoesäuren, Pyridincarbonsäuren u.s.w., spielen in der chemischen und pharmazeutischen Industrie auf verschiedenen Sachgebieten, wie dem Gebiet der Geschmacks- und Geruchsstoffe, dem Farbstoff-, Pflanzenschutz- und Pharmasektor, eine wichtige Rolle als Zwischenprodukte mit besonders hohem Wertschöpfungspotential.
Die Oxidation von alkylaromatischen Verbindungen zur Gewinnung der aromatischen Carbonylverbindungen ist dabei eine wichtige Reaktion in der organischen Chemie, da sie die Umwandlung von leicht zugänglichen bzw. erhältlichen organischen Substraten mit limitierter Reaktivität in Verbindungen mit mehr reaktiven funktionellen Gruppen und somit mit höherer Reaktivität ermöglicht.
Aus diesem Grund wurden bereits mehrere Methoden zur Oxidation von alkylaromatischen Verbindungen vorgeschlagen.
So beschreiben beispielsweise Hanotier J. et. al, J.C.S.Perkin II, (1972), S. 381-386 die Oxidation von Alkylaromaten in Essigsäure, in Anwesenheit von Sauerstoff und einer starken Säure unter Verwendung von Mangan- oder Cobaltacetat als Katalysator. Die Ausbeute an den entsprechenden Carbonsäuren ist dabei nicht zufriedenstellend. Ein Nachteil bei diesem Verfahren ist weiters, dass nicht nur die entsprechenden Aldehyde oder Ketone als Nebenprodukt in hohen Prozentsätzen auftreten, sondern auch noch ein großer Anteil an dem korrespondierenden Acetat und Alkohol erhalten wird.
In J.Org.Chem. (1960); Vol 25, S. 616- 617 beschreiben Hay A. et.al die katalytische Ozon-initiierte Oxidation mittel Sauerstoff von Xylolen unter Verwendung eines Cobaltkatalysators in Essigsäure bei Rückflusstemperatur. Als Produkt wird dabei die entsprechende Methylbenzoesäure erhalten, die zur korrespondierenden Disäure weiteroxidiert, sodass die Ausbeute an Monocarbonsäure deutlich verringert wird.

Wegen des steigenden Bedarfes an aromatischen oder heteroaromatische Carbonsäuren, sowie wegen der verschiedenen Mängel der bekannten Verfahren, wie großer Prozentsatz an Nebenprodukten, Temperaturwahl von beispielsweise Rückflusstemperatur, sehr spezielle Katalysatoren u.s.w., war es Aufgabe der Erfindung ein Verfahren zu finden, das die gewünschten aromatischen und heteroaromatischen Carbonsäuren in höheren Ausbeuten mit geringerer Nebenproduktbildung unter einfachen Verfahrensbedingungen ermöglicht.

Unerwarteterweise konnte diese Aufgabe durch ein Verfahren gelöst werden, bei welchem aromatische und heteroaromatische Carbonsäuren durch Oxidation von alkylaromatischen bzw. -heteroaromatischen Verbindungen mit Ozon bei milden Reaktionsbedingungen unter Verwendung von leicht zugänglichen Katalysatoren in hoher Ausbeute und geringem bis vernachlässigbaren Nebenproduktanteil erhalten werden.

Gegenstand der Erfindung ist demnach ein Verfahren zur katalytischen Oxidation von alkylaromatischen Verbindungen der Formel (I)

Ar-CH₃

in der Ar einen aromatischen oder heteroaromatischen 5er- oder 6er-Ring oder ein aromatisches oder heteroaromatisches Ringsystem mit bis zu 20 C-Atomen bedeutet, wobei Ar gegebenenfalls ein- oder mehrfach durch C₁-C₆-Alkyl oder -Alkoxy, Halogen, NO₂, CN, OH, gegebenenfalls substituiertes Phenyl, gegebenenfalls substituiertes Pyridyl, NR₁R₂, mit R₁ und R₂ unabhängig voneinander H oder C₁-C₄-Alkyl; Ketogruppen, Sulphonsäuregruppen, Sulfonylchlorid, Silylreste, Siloxy oder Siloxansubstituenten substituiert oder mit einer C₁-C₆-Alkylgruppe, bei der bis zu 2 C-Atome durch ein Heteroatom ersetzt werden können, anelliert sein kann,
zu der korrespondierenden aromatischen oder heteroaromatischen Carbonsäure, das dadurch gekennzeichnet ist, dass eine Verbindung der Formel (I) in einem Lösungsmittel aus der Gruppe der C₁-C₆-Mono- oder Dicarbonsäuren, Ester, Wasser, halogenierte Kohlenwasserstoffe, Acetonitril, C₁-C₆-Alkohole, Silicone, Siliconöle, oder chemisch inerte hochsiedende Lösungsmittel oder Gemische davon, mit Ozon in Gegenwart eines Übergangsmetallkatalysators und gegebenenfalls in Gegenwart einer Säure bei einer Temperatur zwischen - 70 °C und +70 °C zu der entsprechenden Carbonsäure oxidiert wird, die sodann aus dem Reaktionsgemisch isoliert wird.

Bei dem erfindungsgemäßen Verfahren werden alkylaromatische bzw. -heteroaromatische Verbindungen der Formel (I) durch katalytische Oxidation in die entsprechende Carbonsäure überführt.
Als Ausgangsverbindungen werden Verbindungen der Formel Ar-CH₃ (I) eingesetzt. Ar bedeutet einen aromatischen oder heteroaromatischen 5er- oder 6er-Ring oder ein aromatisches oder heteroaromatisches Ringsystem mit bis zu 20 C-Atomen.
Beispiele dafür sind Phenyl, Naphthyl, Anthracen, Phenanthren, Inden, Thiophen, Pyrrol, Furan, Imidazol, Pyridin, Pyridazin, Pyrimidin, Pyrazin, Chinolin, Azepin, Phenantridin, Isochinolin, Indol, Dibenzothiophen, Dibenzofuran, beta-Carbolin, Indolizin, Carbazol, Purin, Pteridin, Indazol, Pyrrazol, 1,2,3-Triazol, 1,2,4-Triazol, Tetrazol, Oxazol, Isoxazol, Piperazin, Dithian, Dioxan, Oxazine, Thiazine, Pyridazine, Cinnolin, Phtalazin, 3:4-Benzocinnolin, Chinazolin, Chinoxalin, Phenazin, Phenoxazin, Phenothiazin, Triazine, Tetrazine, u.s.w.
Bevorzugt bedeutet Ar Phenyl, Naphthyl, Pyridin, Imidazol und Chinolin.
Ar kann dabei gegebenenfalls ein- oder mehrfach durch C₁-C₆-Alkyl oder -Alkoxy, Halogen, NO₂, NR₁R₂, mit R₁ und R₂ unabhängig voneinander H oder C₁-C₄-Alkyl; CN, OH, gegebenenfalls substituiertes Phenyl, gegebenenfalls substituiertes Pyridyl, Ketogruppen, Sulphonsäuregruppen, Sulfonylchlorid, Siloxanen, Silylresten, Siloxy substituiert sein.
Unter C₁-C₆-Alkyl oder -Alkoxy sind dabei lineare oder verzweigte Alkyl- oder Alkoxygruppen, wie Methyl, Ethyl, n-Propyl, i-Propyl, n-Butyl, tert.-Butyl, Methoxy, Ethoxy, Propoxy, Butoxy, Hexyloxy u.s.w., zu verstehen. Bevorzugt sind C₁-C₄-Alkyl- und C₁-C₂-Alkoxyreste.
Halogen bedeutet Fluor, Chlor, Brom oder Jod, wobei Fluor, Chlor und Brom bevorzugt sind.
Unter NR₁R₂ sind Amin und substituierte Amine zu verstehen, wobei R₁ und R₂ unabhängig voneinander H oder C₁-C₄-Alkyl bedeuten können. Bevorzugt bedeuten beide Reste R₁ und R₂ H oder C₁-C₂-Alkyl.
Als Silylreste eignen sich beispielsweise Silyl, Trimethylsilyl, Phenyldimetylsilyl, ditert-Butylmethylsilyl.
Ar kann gegebenenfalls auch mit einer C₁-C₆-Alkylgruppe, bei der bis zu 2 C-Atome durch ein Heteroatom ersetzt werden können, anelliert sein.

Falls erforderlich können die Substituenten durch geeignete, aus dem Stand der Technik bekannte Schutzgruppen geschützt werden.

Beispiele für Verbindungen der Formel (I) sind Toluol, 2- Bromtoluol, 3-Bromtoluol, 4-Bromtoluol, 2, Chlortoluol, 3-Chlortoluol, 4-Chlortoluol, 2-Fluortoluol, 3-Fluortoluol, 4-Fluortoluol, 3,4-Dichlortoluol, 3,4-Difluortoluol, 4-tert.-Butyltoluol, Ethylbenzol, o- Nitrotoluol, m-Nitrotoluol, p-Trifluormethyltoluol, 3-5-Di-(Trifluormethyl)toluol, p-Nitrotoluol, p-Toluidin, p-Cumol, Kresole, Mono-, Di- oder Trialkyl- oder alkoxynaphthaline, wie Methylnaphthaline, Methoxynaphthaline, Methylanthracen, Diphenylmethan, p-Toluolsulfonylchlorid, 2-Methylpyridin, 3-Methylpyridin, 4-Methylpyridin, 2,3-Dimethylpyridin, 2,4-Dimethylpyridin, 2,5-Di-methylpyridin, 2,6-Dimethylpyridin, 3,5-Di-tert-Butyl-4-hydroxytoluol, 3,4-Di-methoxytoluol, 2,3-Dihydroxytoluol, 3,4,5-Trimethoxytoluol, 2-Hydroxy-5-nitrotoluol, 3,4-Dihydroxy-5-nitrotoluol, 4-Cyanotoluol, 2-(p-Tolyl)-pyridin, 4-(p-Tolyl)-pyridin, u.s.w.

Die erfindungsgemäße katalytische Oxidation erfolgt in einem Lösungsmittel aus der Gruppe der C₁-C₆-Mono- oder Dicarbonsäuren, Ester, Wasser, halogenierte Kohlenwasserstoffe, Silicone, Siliconöle, chemisch inerte hochsiedende Lösungsmittel, Acetonitril, Aceton oder C₁-C₆-Alkohole.
Geeignete Lösungsmittel sind demnach C₁-C₆-Mono- oder Dicarbonsäuren, wie etwa Essigsäure, Propionsäure, Buttersäure, u.s.w., sowie Lösungen von Carbonsäuren in anderen Lösungsmitteln.
Als Ester werden bevorzugt C₁-C₆-Carbonsäureester, wie etwa Ethylacetat, Isopropylacetat, t-Butylacetat, n- Butylacetat, eingesetzt.
Als halogenierte Kohlenwasserstoffe eignen sich beispielsweise Dichlormethan, 1,2-Dichlorethan, Chloroform oder Tetrachlorkohlenstoff.
Weiters eignen sich C₁-C₆-Alkohole, wie etwa Methanol, n-Butanol, tert.-Butanol, Silicone und Siliconöle und chemisch inerte hochsiedende Lösungsmittel z.B. Weißöl.
Oben genannte Lösungsmittel können auch als Gemische eingesetzt werden.

Bevorzugt werden C₁-C₄-Mono- oder Dicarbonsäuren wie etwa Essigsäure, Propionsäure, Buttersäure oder C₁-C₄-Alkohole, wie etwa Methanol, n-Butanol, tert.-Butanol; oder Gemische mit Wasser oder chlorierten Kohlenwasserstoffen eingesetzt.
Besonders bevorzugte Lösungsmittel sind Essigsäure, oder Mischungen von Essigsäure in Wasser oder nur Wasser

Als Katalysator werden Übergangsmetallkatalysatoren eingesetzt.
Geeignete Übergangsmetallkatalysatoren basieren auf den Elementen aus der Gruppe 3B, wie Sc, Y, La, Ce, Sm oder Lanthanoiden, Ac oder andere Actinoiden, aus der Gruppe 4B (Ti, Zr, Hf), Gruppe 5B (V, Nb, Ta), Gruppe 6B (Cr, Mo, W), Gruppe 7B (Mn, Tc, Re), Gruppe 8 (Fe, Ru, Os, Co, Rh, Ir, Ni, Pd, Pt), Gruppe 1B (Cu, Ag, Au).
Bevorzugt werden Katalysatoren eingesetzt, die Sc, Sm oder Y aus der Gruppe 3B, La oder Ce aus der Gruppe der Lanthaniden; Ti, Zr aus der Gruppe 4B, V oder Ta aus der Gruppe 5B, Cr, Mo oder W aus der Gruppe 6B, Mn oder Re aus der Gruppe 7B, Fe, Co, Ni, Pd aus der Gruppe 8B oder Cu aus der Gruppe 1B enthalten.

Besonders bevorzugt sind Katalysatoren, die Sm, La, Ce, Ni, Co, V, Cr, Fe, Mn oder Cu enthalten.
Der Katalysator kann dabei als Metallhydroxid, Metalloxid, als organisches Salz, anorganisches Salz, u.s.w. eingesetzt werden.
Hydroxyde sind beispielsweise Mn(OH)₂, MnO(OH), Fe(OH)₂ und Fe(OH)₃. Geeignete Oxide sind beispielsweise Sm₂O₃, TiO₂, ZrO₂, V₂O₃, V₂O₅, CrO, Cr₂O₃, MoO₃, MnO, Mn₃O₄, Mn₂O₃, MnO₂, Mn₂O₇, FeO, Fe₂O₃, Fe₃O₄, RuO₂, RuO₄, CoO, CoO₂, Co₂O₃, Cu₂O₃, u.s.w.
Organische Salze sind beispielsweise Formiate, Acetate, Propionate, Acetylacetonate, Benzoate, Alkoholate, Naphthenate, Stearate und andere Salze mit C₂-C₂₀ Fettsäuren von Co, Mn, Ce, Ti, Zr, V, Cr, Mo, Fe, Ru, Ni, Pd, Cu u.s.w..
Anorganische Salze beinhalten beispielsweise Nitrate, Sulfate, Phosphate, Carbonate und Halogenide von V, Co, Fe, Mn, Ni, Cu, u.s.w.
Der Katalysator kann auch in Form eines Komplexes, wie etwa in EP-A1-0 824 962 beschrieben, als 2-kerniger Katalysator, mit Liganden oder verbrückt, u.s.w. eingesetzt werden.
Bevorzugt wird der Katalysator als organisches Salz, z.B. Acetat, Acetylacetonat, Picolinat, Citrat, Neucuproin u.s.w., sowie als anorganisches Salz, z.B. Nitrat u.s.w. eingesetzt.
Der Katalysator kann gewünschtenfalls in fester Form auf einen Träger aufgebracht sein. Geeignete Träger sind beispielsweise Silica, Zeolith, Aktivkohle, u.s.w. oder andere poröse Träger. Bei dem erfindungsgemäßen Verfahren können auch Katalysatormischungen aus 2 oder mehreren Katalysatoren eingesetzt werden.
Bei dem erfindungsgemäßen Verfahren wird der Katalysator bzw. die Katalysatormischung in einem Menge von 0,001 mol% bis 1 mol%, bevorzugt von 0,01 bis 1 mol% und besonders bevorzugt von 0,1 bis 0,25 mol % pro mol Substrat eingesetzt.

Die katalytische Oxidation erfolgt gegebenenfalls in Anwesenheit einer Säure. Als Säure kommen starke Säuren, wie etwa H₂SO₄, H₂SO₄/SO₃, HNO₃ sowie andere Mineralsäuren oder Trichloressigsäure, Trifluoressigsäure, Metansulfonsäure oder andere Sulfonsäuren in Frage.
Bevorzugt wird das erfindungsgemäße Verfahren in Anwesenheit einer Säure durchgeführt, wobei H₂SO₄ als Säure besonders bevorzugt wird.
Die Menge an zugesetzter Säure beträgt 0,001 mol bis 1 mol, bevorzugt 0,01 bis 0,8 mol und besonders bevorzugt 0,1 bis 0,5 mol pro mol Substrat.

Als Oxidationsmittel dient Ozon. Ozon wird dabei bevorzugt in etwa 2-facher äquimolarer Menge, bezogen auf das Substrat eingesetzt. Es kann jedoch auch ein Überschuss an Ozon verwendet werden.

Die Reaktionstemperatur liegt bei dem erfindungsgemäßen Verfahren zwischen -70 und +70°C, bevorzugt zwischen -20 und 70°C und besonders bevorzugt zwischen 0 und 30°C.

Bei dem erfindungsgemäßen Verfahren werden das Substrat, der Katalysator und gegebenenfalls die Säure in dem entsprechenden Lösungsmittel vorgelegt und auf die gewünschte Reaktionstemperatur gebracht. Anschließend wird Ozon in das Reaktionsgemisch eingeleitet, bis die Ozonolyse beendet ist. Gegebenenfalls im Lösungsmittel verbleibendes, überschüssiges Ozon wird mit Stickstoff ausgeblasen.

Die Isolierung des Reaktionsproduktes erfolgt sodann in Abhängigkeit von dessen Aggregatzustand durch übliche Methoden, wie Extraktion, Destillation, Chromatographie, u.s.w. bei flüssigen Produkten oder Filtration, Zentrifugation, Sublimation u.s.w. bei festen Produkten.

Durch das erfindungsgemäße Verfahren werden die gewünschten aromatischen und heteroaromatischen Carbonsäuren in hohen Ausbeuten, mit im Vergleich zum Stand der Technik wesentlich geringerem Nebenproduktanteil, auf einfache Weise erhalten.

Besonders geeignet ist das erfindungsgemäße Verfahren zur Herstellung von substituierter Benzoesäure, Pyridincarbonsäuren, Naphthalincarbonsäuren oder anderen heteroaromatischen Carbonsäuren.

Beispiele für Carbonsäuren, die erfindungsgemäß in vorteilhafter Weise hergestellt werden können sind:
2-Brombenzoesäure, 3-Brombenzoesäure, 4-Brombenzoesäure, 2,3-Dibrombenzoesäure, 2,4-Dibrombenzoesäure, 2,6-Dibrombenzoesäure, 3,4-Dibrombenzoesäure, 3,5-Dibrombenzoesäure, 2,4,6-Tribrombenzoesäure, 2,3,5-Tribrombenzoesäure, 2,3,6-Tribrombenzoesäure, 2-Fluorbenzoesäure, 3-Fluorbenzoesäure, 4-Fluorbenzoesäure, 2,3-Difluorbenzoesäure, 2,4-Difluorbenzoesäure, 2,6-Difluorbenzoesäure, 3,4-Difluorbenzoesäure, 3,5-Difluorbenzoesäure, 2,4,6-Trifluorbenzoesäure, 2,3,5-Trifluorbenzoesäure, 2,3,6-Trifluorbenzoesäure, 2-Chlorbenzoesäure, 3-Chlorbenzoesäure, 4-Chlorbenzoesäure, 2,3-Dichlorbenzoesäure, 2,4-Dichlorbenzoesäure, 2,6-Dichlorbenzoesäure, 3,4-Dichlorbenzoesäure, 3,5-Dichlorbenzoesäure, 2,4,6-Trichlorbenzoesäure, 2,6-Dichlorbenzoesäure, 2,3,5-Trichlorbenzoesäure, 2,3,6-Trichlorbenzoesäure, 2-lodbenzoesäure, 3-lodbenzoesäure, 4-lodbenzoesäure, 2,3-Diiodbenzoesäure, 2,4-Diiodbenzoesäure, 2,6-Diiodbenzoesäure, 3,4-Diiodbenzoesäure, 3,5-Diiod-benzoesäure, 2,4,6-Triiodbenzoesäure, 2,6-Diiodbenzoesäure, 2,3,5-Triiod-benzoesäure, 2,3,6-Triiod-benzoesäure, 2-Methoxybenzoesäure, 3-Methoxy-benzoesäure, 4-Methoxybenzoesäure, 2,3-Dimethoxybenzoesäure, 2,4-Dimethoxybenzoesäure, 2,6-Dimethoxybenzoesäure, 3,4-Di-Methoxybenzoesäure, 3,5-Dimethoxybenzoesäure, 2,4,6-Trimethoxybenzoesäure, 2,6-Dimethoxybenzoesäure, 2,3,5-Trimethoxybenzoesäure, 2,3,6-Trimethoxybenzoesäure, 2-Ethoxybenzoesäure, 3-Ethoxybenzoesäure, 4-Ethoxybenzoesäure, 2,3-Diethoxybenzoesäure, 2,4-Diethoxybenzoesäure, 2,6-Diethoxybenzoesäure, 3,4-Diethoxybenzoesäure, 3,5-Diethoxybenzoesäure, 2,4,6-Triethoxybenzoesäure, 2,3,5-Triethoxybenzoesäure, 2,3,6-Triethoxybenzoesäure, 2-Nitrobenzoesäure, 3-Nitrobenzoesäure, 4-Nitrobenzoesäure, 2,3-Dinitrobenzoesäure, 2,4-Dinitrobenzoesäure, 2,6-Dinitrobenzoesäure, 3,4-Dinitrobenzoesäure, 3,5-Dinitrobenzoesäure, 2,4,6-Trinitrobenzoesäure, 2,3,5-Trinitrobenzoesäure, 2,3,6-Trinitrobenzoesäure, 2-Cyano-benzoesäure, 3-Cyanobenzoesäure, 4-Cyanobenzoesäure, 2,3-Dicyanobenzoesäure, 2,4-Dicyanobenzoesäure, 2,6-Dicyanobenzoesäure, 3,4-Dicyanobenzoesäure, 3,5-Dicyanobenzoesäure, 2,4,6-Tricyanobenzoesäure, 2,3,5-Tricyanobenzoesäure, 2,3,6-Tricyanobenzoesäure, 2-Trifluormethylbenzoesäure, 3-Trifluormethylbenzoesäure, 4-Trifluormethylbenzoesäure, 2,3-Di-trifluormethylbenzoesäure, 2,4-Di-trifluormethylbenzoesäure, 2,6-Di-trifluormethylbenzoesäure, 3,4-Di-Trifluormethylbenzoesäure, 3,5-Di-Trifluormethylbenzoesäure, 2,4,6-Tri-Trifluormethylbenzoesäure, 2,3,5-Tri-Trifluormethylbenzoesäure, 2,3,6-Tri-Trifluormethylbenzoesäure, 2-Methylbenzoesäure, 3-Methylbenzoesäure, 4-Methylbenzoesäure, 2,3-Dimethylbenzoesäure, 2,4-Dimethylbenzoesäure, 2,6-Di-Methylbenzoesäure, 3,4-Dimethylbenzoesäure, 3,5-Dimethylbenzoesäure, 2,4,6-Trimethylbenzoesäure, 2,3,5-Trimethylbenzoesäure, 2,3,6-Trimethylbenzoesäure, 2-Ethylbenzoesäure, 3-Ethylbenzoesäure, 4-Ethylbenzoesäure, 2,3-Diethylbenzoesäure, 2,4-Diethylbenzoesäure, 2,6-Diethylbenzoesäure, 3,4-Diethylbenzoesäure, 3,5-Diethylbenzoesäure, 2,4,6-Triethylbenzoesäure, 2,3,5-Triethylbenzoesäure, 2,3,6-Triethylbenzoesäure, 2-Aminobenzoesäure, 3-Aminobenzoesäure, 4-Aminobenzoesäure, 2,3-Diaminobenzoesäure, 2,4-Diaminobenzoesäure, 2,6-Diaminobenzoesäure, 3,4-Diaminobenzoesäure, 3,5-Diaminobenzoesäure, 2,4,6-Triaminobenzoesäure, 2,3,5-Triaminobenzoesäure, 2,3,6-Triaminobenzoesäure, 4-tert-Butylbenzoesäure, 3-tert-Butylbenzoesäure, 3,5-Di-tert-Butylbenzoesäure, 4-Hydroxy-3,5-di-tert-Butylbenzoesäure, 4-Fluor-3,5-Di-tert-Butylbenzoesäure, 2-Chlor-4-tert-Butylbenzoesäure, 2-Fluor-Ditert-Butyl-benzoesäure, 4-Hydroxy-3,5-di-Nitrobenzoesäure, 4-Fluor-3,5-Di-Nitrobenzoesäure, 2-Chlor-4-Nitrobenzoesäure, 2-Fluor-Di-Nitrobenzoesäure, 2-Hydroxy-5-Nitrobenzoesäure, 3-Nitro-5-Trifluormethylbenzoesäure, 4-Hydroxy-3,5-di-Brombenzoesäure, 4-Fluor-3,5-Di-Brombenzoesäure, 2-Chlor-4-Brombenzoesäure, 2-Fluor-Di-Brom-benzoesäure, 2-Hydroxy-5-Brombenzoesäure, 3-Brom-5-Trifluormethylbenzoesäure, 4-Hydroxy-3,5-di-Chlorbenzoesäure, 4-Fluor-3,5-Di-Chlorbenzoesäure, 2-Fluor-Di-Chlorbenzoesäure, 2-Hydroxy-5-Chlorbenzoesäure, 3-Chlor-5-Trifluormethylbenzoesäure, 4-Hydroxy-3,5-di-Fluorbenzoesäure, 2-Chlor-4-Fluorbenzoesäure, 2-Hydroxy-5-Fluorbenzoesäure, 3-Fluor-5-Trifluormethylbenzoesäure, 4-(2-Pyridyl)-benzoesäure, 4-(4-Pyridyl)-benzoesäure, 2-Pyridincarbonsäure, 3-Pyridincarbonsäure, 4-Pyridincarbonsäure u.s.w..

### Beispiel 1: 4-Brombenzoesäure

In einem 100 ml Doppelmantelgefäß wurden 200 ml Essigsäure, 0,24 g (0,89 mmol) Mangan(II)acetat, 9,8 g (0,1 mol) Schwefelsäure und 34,1 g (0,2 mol) 4-Bromtoluol vorgelegt. Es wurde auf 16 °C abgekühlt und 20,0 g (0,41 mol) Ozon in einem Zeitraum von 75 Minuten eingeleitet.
Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 4-Bromtoluol: < 0,1 %; 4-Brombenzoesäure: 98%

### Beispiel 2: 4-Brombenzoesäure

In einem 100 ml Doppelmantelgefäß wurden 200 ml Essigsäure, 0,24 (0,98) g Mangan(III)acetat, und 34,1 g (0,2 mmol) 4-Bromtoluol vorgelegt. Es wurde auf 16 °C abgekühlt und 20,0 g (0,41 mol) Ozon in einem Zeitraum von 100 Minuten eingeleitet.
Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 4-Bromtoluol: < 0,1 %, 4-Brombenzoesäure: 98%

### Beispiel 3: 4-tert-Butylbenzoesäure

In einem 100 ml Doppelmantelgefäß wurden 200 ml Essigsäure, 0,24 g Mangan(II)acetat, 9,8 g Schwefelsäure und 14,8 g 4-tert-Butyltoluol vorgelegt. Es wurde auf 16 °C abgekühlt und 10,5 g Ozon in einem Zeitraum von 100 Minuten eingeleitet.

Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 4-tert-Butyltoluol: < 0,1 %, 4-tert-Butylbenzoesäure: 98%

### Beispiel 4: 3,4-Dichlorbenzoesäure

In einem 100 ml Doppelmantelgefäß wurden 200 ml Essigsäure, 0,24 g Mangan(II)acetat, 9,8 g Schwefelsäure und 34,5 g 3,4-Dichlortoluol vorgelegt. Es wurde auf 16 °C abgekühlt und 20 g Ozon in einem Zeitraum von 175 Minuten eingeleitet. Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 3,4-Dichlortoluol: < 0,1 %, 3,4-Dichlorbenzoesäure: 96%

### Beispiel 5: 3,4-Difluorbenzoesäure

In einem 100 ml Doppelmantelgefäß wurden 200 ml Essigsäure, 0,05 g Mangan(II)acetat, 1,91 g Schwefelsäure und 14,1 g 3,4-Difluortoluol vorgelegt. Es wurde auf 16 °C abgekühlt und 4,0 g Ozon in einem Zeitraum von 60 Minuten eingeleitet. Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 3,4-Difluortoluol: < 0,1 %, 3,4-Difluorbenzoesäure: 98%

### Beispiel 6: 3-Chlorbenzoesäure

In einem 100 ml Doppelmantelgefäß wurden 200 ml Essigsäure, 0,08 g Cer(III)acetat, 4,9 g Schwefelsäure und 12,65 g 3-Chlortoluol vorgelegt. Es wurde auf 16 °C abgekühlt und 5,15 g Ozon in einem Zeitraum von 75 Minuten eingeleitet.
Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 3-Chlortoluol: < 0,1 %, 3-Chlorbenzoesäure: 98%

### Beispiel 7: 2-Pyridincarbonsäure

In einem 100 ml Doppelmantelgefäß wurden 200 ml Essigsäure, 0,05 g Mangan(II)acetat, 1,91 g Schwefelsäure und 14,1 g 2-Methylpyridin vorgelegt. Es wurde auf 16 °C abgekühlt und 4,0 g Ozon in einem Zeitraum von 60 Minuten eingeleitet. Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 2-Methylpyridin: < 0,1 %, 2-Pyridincarbonsäure: 97%

### Beispiel 8: 3-Pyridincarbonsäure

In einem 100 ml Doppelmantelgefäß wurden 200 ml Essigsäure, 0,05 g Mangan(II)acetat, 1,91 g Schwefelsäure und 14,1 g 3-Methylpyridin vorgelegt. Es wurde auf 16 °C abgekühlt und 4,0 g Ozon in einem Zeitraum von 60 Minuten eingeleitet. Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 3-Methylpyridin: < 0,1 %, 3-Pyridincarbonsäure: 96-98%

### Beispiel 9: 4-Pyridincarbonsäure

In einem 100 ml Doppelmantelgefäß wurden 200 ml Essigsäure, 0,05 g Mangan(II)acetat, 1,91 g Schwefelsäure und 14,1 g 4-Methylpyridin vorgelegt. Es wurde auf 16 °C abgekühlt und 4,0 g Ozon in einem Zeitraum von 60 Minuten eingeleitet. Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 4-Methylpyridin: < 0,1 %, 4-Pyridincarbonsäure: 97%

### Beispiel 10: 4-(2-Pyridyl)-benzoesäure

In einem 100 ml Doppelmantelgefäß wurden 200 ml Essigsäure, 0,05 g Mangan(II)acetat, 1,74 g Schwefelsäure und 3,0g g 2-(p-Tolyl)-pyridin vorgelegt. Es wurde auf 16 °C abgekühlt und 4,0 g Ozon in einem Zeitraum von 60 Minuten eingeleitet.
Nach beendeter Ozonolyse wurde mit Stickstoff das im Lösungsmittel vorhandene Ozon ausgeblasen.

### Analyse des Reaktionsgemisches mittels HPLC oder GC ergab folgende Resultate: 2-(p-Tolyl)-pyridin : < 0,1 %, 4-(2-Pyridyl)-benzoesäure: 94-97%

### Beispiel 11: 4-(4-Pyridyl)-benzoesäure

In einem 100 ml Doppelmantelgefäß wurden 200 ml Essigsäure, 0,05 g Mangan(II)acetat, 1,74 g Schwefelsäure und 3,0g g 4-(p-Tolyl)-pyridin vorgelegt. Es wurde auf 16 °C abgekühlt und 4,0 g Ozon in einem Zeitraum von 60 Minuten eingeleitet.
Nach beendeter Ozonolyse wurde mit Stickstoff das im Lösungsmittel vorhandene Ozon ausgeblasen.

Analyse des Reaktionsgemisches mittels HPLC oder GC ergab folgende Resultate: 4-(p-Tolyl)-pyridin : < 0,1 %, 4-(4-Pyridyl)-benzoesäure: 94-97%

## Patentansprüche

1. Verfahren zur katalytischen Oxidation von alkylaromatischen Verbindungen der Formel (I)
Ar-CH₃
in der Ar einen aromatischen oder heteroaromatischen 5er- oder 6er-Ring oder ein aromatisches oder heteroaromatisches Ringsystem mit bis zu 20 C-Atomen bedeutet, wobei Ar gegebenenfalls ein- oder mehrfach durch C₁-C₆-Alkyl oder -Alkoxy, Halogen, NO₂, NR₁R₂ mit R₁ und R₂ unabhängig voneinander H oder C₁-C₄-Alkyl, CN, OH, gegebenenfalls substituiertes Phenyl, gegebenenfalls substituiertes Pyridyl, Ketogruppen, Sulphonsäuregruppen, Sulfonylchlorid, Silylreste, Siloxy oder Siloxansubstituenten substituiert oder mit einer C₁-C₆-Alkylgruppe, bei der bis zu 2 C-Atome durch ein Heteroatom ersetzt werden können, anelliert sein kann,
zu den korrespondierenden aromatischen oder heteroaromatischen Carbonsäuren, **dadurch gekennzeichnet, dass** eine Verbindung der Formel (I) in einem Lösungsmittel aus der Gruppe der C₁-C₆-Mono- oder Dicarbonsäuren, Ester, Wasser, halogenierte Kohlenwasserstoffe, Acetonitril, C₁-C₆-Alkohole, Silicone, Siliconöle, oder chemisch inerte hochsiedende Lösungsmittel oder Gemische davon, mit Ozon in Gegenwart eines Übergangsmetallkatalysators und gegebenenfalls in Gegenwart einer Säure bei einer Temperatur zwischen - 70 °C und +70 °C zu der entsprechenden Carbonsäure oxidiert wird, die sodann aus dem Reaktionsgemisch isoliert wird.

2. Verfahren nach Anspruch 1, **dadurch gekennzeichnet, dass** in der Formel (I) Ar einen Phenyl-, Naphthyl-, Anthracen-, Pyridin-, Imidazol- oder Chinolinring bzw. - ringsystem bedeutet, das gegebenenfalls ein- oder mehrfach durch C₁-C₄-Alkyl, C₁-C₂-Alkoxy, Fluor, Chlor, Brom, lod, NO₂, OH, CN, gegebenenfalls substituiertes Phenyl, gegebenenfalls substituiertes Pyridyl oder NH₂ substituiert oder mit einer C₄-Alkylgruppe, bei der gegebenenfalls zwei C-Atome durch O ersetzt sein könne, anelliert sein kann.

3. Verfahren nach Anspruch 1, **dadurch gekennzeichnet, dass** als Lösungsmittel Essigsäure, Propionsäure, Buttersäure, Methanol, n-Butanol, tert. Butanol oder Gemische mit Wasser oder einem chlorierten Kohlenwasserstoff eingesetzt werden.

4. Verfahren nach Anspruch 1, **dadurch gekennzeichnet, dass** als Übergangsmetallkatalysator ein Katalysator basierend auf einem der Elemente Sc, Sm oder Y aus der Gruppe 3B, La oder Ce aus der Gruppe der Lanthaniden; Ti, Zr aus der Gruppe 4B, V oder Ta aus der Gruppe 5B, Cr, Mo oder W aus der Gruppe 6B, Mn oder Re aus der Gruppe 7B, Fe, Co, Ni, Pd aus der Gruppe 8B oder Cu aus der Gruppe 1B in Form eines organischen oder anorganischen Salzes, oder ein Gemisch derselben eingesetzt wird.

5. Verfahren nach Anspruch 1, **dadurch gekennzeichnet, dass** die katalytische Oxidation in Anwesenheit einer starken Säure aus der Gruppe H₂SO₄, H₂SO₄/SO₃, HNO₃, Trichloressigsäure, Trifluoressigsäure oder Metansulfonsäure durchgeführt wird.

6. Verfahren nach Anspruch 1, **dadurch gekennzeichnet, dass** die katalytische Oxidation bei einer Temperatur zwischen -20°C und +70°C durchgeführt wird.

7. Verfahren nach Anspruch 1, **dadurch gekennzeichnet, dass** Ozon in 2-facher äquimolarer Menge bezogen auf das Substrat eingesetzt wird.

8. Verfahren nach Anspruch 1, **dadurch gekennzeichnet, dass** nach beendeter Ozonolyse gegebenenfalls im Lösungsmittel verbleibendes Ozon mit Stickstoff ausgeblasen wird und die Carbonsäure in Abhängigkeit vom Aggregatzustand durch Extraktion, Destillation, Chromatographie, Filtration, Zentrifugation oder Sublimation aus dem Reaktionsgemisch isoliert wird.
